Europäisches Patentamt

European Patent Office

Office européen des brevets

(19)

(11) Numéro de publication : **0 021 953**

**B1**

(12)

# FASCICULE DE BREVET EUROPÉEN

(45) Date de publication du fascicule du brevet :
**18.09.85**

(21) Numéro de dépôt : **80400847.2**

(22) Date de dépôt : **11.06.80**

(51) Int. Cl.⁴ : **A 61 K   9/00,** A 61 K 31/505

(54) Solutions aqueuses stables d'alloxane.

(30) Priorité : **15.06.79 FR 7915363**

(43) Date de publication de la demande :
**07.01.81 Bulletin 81/01**

(45) Mention de la délivrance du brevet :
**18.09.85 Bulletin 85/38**

(84) Etats contractants désignés :
**BE CH DE GB IT LI NL**

(56) Documents cités :
**CH-A-   277 825**
**FR-M-     3 743**
**CHEMICAL ABSTRACTS, vol. 51, no. 16, 25 août 1957,
colonne 13193e Columbus, Ohio, US J. DUFRENOY:
"Chemotherapy of cancer, using alloxan"**
**THE MERCK INDEX, éd. 8, publié par Merck et Co.
Inc. 1968, page 993 Rahway, New Jersey US**
**CHEMICAL ABSTRACTS, vol. 82, no. 2 13 janvier
1975, page 218, colonne 2, réf. 7659y Columbus,
Ohio, US**
**CHEMICAL ABSTRACTS, vol. 83, no. 20 17 novembre
1975, page 268, colonne 1, réf. 16380m Columbus,
Ohio, US M. MARTIN et al.: "Streptokinase stability
pattern during storage in various solvents and at
different temperatures"**
**Le dossier contient des informations techniques présentées postérieurement au dépôt de la demande et
ne figurant pas dans le présent fascicule.**

(73) Titulaire : **Griffon, Henri**
**72, Rue de Longchamps**
**F-75782 Paris Cedex 16 (FR)**

(72) Inventeur : **Griffon, Henri**
**72, Rue de Longchamps**
**F-75782 Paris Cedex 16 (FR)**

(74) Mandataire : **Petit, Hélène et al**
**Cabinet Hélène Petit 94, Avenue Kléber**
**F-75116 Paris (FR)**

## Description

La présente invention concerne un procédé de préparation d'une solution aqueuse d'alloxane stable.

On sait en effet, que l'alloxane qui se présente en poudre sous forme anhydre, monohydratée ou tétrahydratée, est facilement soluble dans l'eau. Mais l'alloxane et ses solutions sont très fragiles et s'altèrent à l'air, à la lumière et à toute augmentation de température. Il est donc extrêmement difficile de les conserver correctement.

L'objet de l'invention est de fournir des solutions aqueuses d'alloxane stables, susceptibles d'être manipulées et conservées dans altération. Cet objet de l'invention est utile en soi pour la progression des techniques de préparation et de conservation des produits chimiques. Il trouve en outre, une application particulièrement intéressante dans la préparation des solutions d'alloxane destinées à l'usage thérapeutique.

On sait en effet que l'alloxane est utilisé avec succès dans la lutte contre le cancer d'après les principes du docteur Pierre Grobon, créateur de l'alloxanothérapie anticancéreuse.

L'administration de l'alloxane se fait exclusivement par voie intramusculaire, de sorte que le médicament doit se présenter sous forme d'ampoules injectables.

Jusqu'à présent, la réalisation de ces ampoules se faisait par préparation de doses fractionnées d'alloxane en solution, lyophilisation et introduction de la poudre ainsi obtenue dans une ampoule que l'on fermait sous atmosphère d'azote pour chasser l'air. Au moment de l'administration, on réalisait un mélange extemporané de la poudre et de quelques millilitres de sérum physiologique. Ce procédé de préparation était particulièrement onéreux et ne donnait pas toute satisfaction, puisque le résidu sec préparé dans ces conditions présentait souvent une coloration rose, témoin de l'existence d'une altération de l'alloxane.

Le nouveau procédé, selon l'invention, permet d'éliminer ces inconvénients et met à la disposition du médecin une solution stable d'alloxane directement injectable et susceptible d'être conservée.

Le procédé de préparation selon la présente invention est caractérisé en ce que l'on prépare une solution d'alloxane dans l'eau et que l'on ajoute, à la quantité en poids d'alloxane pur présente dans la solution, un sucre réducteur dans une quantité en poids de 5 à 25 fois plus grande que ladite quantité d'alloxane.

Le sucre réducteur peut être du glucose, du lactose et préférentiellement du lévulose qui est notamment le plus soluble des trois sucres. On met ainsi à profit le fait que, l'alloxane étant un réducteur, très oxydable même par l'oxygène de l'air, son potentiel réducteur est augmenté si on additionne le milieu d'un réducteur ; et partant la protection du produit en est d'autant augmentée. S'agissant d'un domaine d'utilisation thérapeutique, il a été trouvé particulièrement avantageux d'utiliser comme réducteurs adjuvants, des sucres réducteurs, notamment ceux précités, qui sont des substances biologiques, d'une inocuité évidente.

Il est connu de « The Merck Index, 8ᵉ édition, 1968, Merck & Co., Inc. Rahvay, N.J., U.S.A., page 593, paragraphe « sucrose » » d'utiliser le sucre inverti comme stabilisant et antioxydant en pharmacie.

On peut mettre en évidence l'action protectrice du sucre réducteur en effectuant le test suivant :

Lorsque l'on ajoute de l'ammoniaque à la solution mixte d'alloxane et de sucre réducteur ainsi préparée, cette dernière reste incolore. Ceci prouve que l'alloxane reste stable et se trouve protégé contre toute altération. En effet, l'addition d'ammoniaque à une solution d'alloxane simple entraîne l'apparition d'une couleur rose, témoin de la dégradation de l'alloxane par l'air et les conditions ambiantes.

Dans le cas particulier de la préparation d'ampoules de solutions d'alloxane pour l'administration thérapeutique, le procédé selon l'invention est le suivant :

On introduit dans du sérum physiologique ou de l'eau pour solutions injectables une quantité prédéterminée d'alloxane et on ajoute une quantité de 5 à 25 fois plus grande d'un sucre réducteur. La stérilisation de la solution peut se faire après la mise en ampoule, de 5 ml et fermeture de ces ampoules, par stérilisation par tyndallisation à 60-70 °C.

Le mode préféré de l'invention consiste à réaliser la stérilisation de la solution, avant la mise en ampoule, en procédant à une filtration sur membrane filtrante utilisée en bactériologie, à température ambiante.

On remplit et on scelle les ampoules en atmosphère stérile.

La conservation des ampoules est effectuée à 40 °C maximum.

La stérilisation directe par autoclave à 120 °C entraîne le jaunissement de la solution contenue dans l'ampoule, ce qui dénote une altération à cette température élevée malgré la présence du sucre protecteur.

On choisit avantageusement une quantité de sucre réducteur égale à environ 5 à 15 fois la quantité d'alloxane anhydre introduite dans la solution.

Les exemples suivants ont pour but d'illustrer la présente préparation d'ampoules injectables.

1. On réalise une solution d'alloxane en introduisant par millilitre de sérum physiologique :
— 10 mg d'alloxane pur anhydre ou bien 12,27 mg d'alloxane monohydraté ou bien 15,07 mg d'alloxane tétrahydraté,
— 150 mg de lévulose.

Si besoin est, on filtre la solution, puis on la soumet à une filtration sur membrane filtrante à la température ambiante. On remplit une ampoule de 5 ml avec 4,5 à 4,6 ml du soluté ainsi préparé et on la scelle, en atmosphère stérile.

Une telle ampoule permet d'y puiser à l'aide d'une seringue à injection montée d'une aiguille appropriée aux injections intramusculaires 4 ml de solution contenant 40 mg d'alloxane qui est la dose optimum unitaire d'utilisation de ce produit.

On peut augmenter la quantité de lévulose mais ne pas dépasser 250 mg par millilitre car on risque un phénomène de charbonnage du sucre au moment de la fermeture de l'ampoule.

2. On réalise une solution d'alloxane en introduisant par millilitre de sérum physiologique :
— 10 mg d'alloxane pur,
— 200 mg de glucose.

On filtre et on conditionne en ampoules stériles comme dans l'exemple précédent.

3. On réalise une solution d'alloxane en introduisant par millilitre de sérum physiologique :
— 10 mg d'alloxane pur,
— 250 mg de lactose.

On peut conditionner en ampoules stériles si désiré, comme dans les exemples précédents.

4. On réalise une solution d'alloxane en introduisant par millilitre d'eau pour solutions injectables :
— 10 mg d'alloxane pur anhydre ou bien 12,27 mg d'alloxane monohydraté, ou bien 15,07 mg d'alloxane tétrahydraté,
— 50 mg de lévulose (ce qui correspond à une solution isotonique de lévulose).

On filtre la solution obtenue sur membrane filtrante à la température ambiante.

On remplit une ampoule de 5 ml et on la scelle en atmosphère stérile.

Cette ampoule contient 50 mg d'alloxane et 250 mg de lévulose. Les ampoules sont conservées à 4 °C maximum et de préférence à l'obscurité.

Le contrôle de l'absence d'altération de l'alloxane a été étudié grâce à la spectrophotométrie en lumière ultra-violette.

On sait qu'en milieu alcoolique l'alloxane donne un spectre d'absorption en lumière ultra-violette, caractérisé par un maximum à 272 millimicrons et un minimum à 262 millimicrons.

Des mesures comparatives ont été réalisées sur des solutions d'alloxane préparées selon le procédé de l'invention (avec sucre réducteur et notamment le lévulose) et sur des solutions contenant la même quantité d'alloxane mais en sérum physiologique sans lévulose.

On a établi les courbes spectrales de l'alloxane dans ces 2 types de solutions en fonction du temps.

Il a été ainsi constaté que seules les solutions dans lesquelles l'alloxane est associée au lévulose sont d'une stabilité remarquable pendant au moins 1 an.

## Revendications

1. Solutions aqueuses stables d'alloxane caractérisées en ce qu'elles contiennent par ml de sérum physiologique ou d'eau pour solutions injectables, une quantité prédéterminée d'alloxane et une quantité 5 à 25 fois plus grande d'un sucre réducteur choisi parmi le groupe constitué par le lévulose, le glucose et le lactose.

2. Solutions aqueuses stables d'alloxane selon la revendication 1 caractérisées en ce qu'elles contiennent par ml de sérum physiologique ou d'eau pour solutions injectables, 10 mg d'alloxane et 50 mg de lévulose.

3. Procédé de préparation de solutions aqueuses d'alloxane stables, caractérisé en ce que l'on dissout dans du sérum physiologique ou de l'eau pour solutions injectables une quantité prédéterminée d'alloxane et qu'on ajoute à cette solution un sucre réducteur dans une quantité en poids 5 à 25 fois plus grande que ladite quantité d'alloxane, le sucre réducteur étant choisi dans le groupe constitué par le lévulose, le glucose et le lactose.

4. Procédé de préparation de solutions aqueuses d'alloxane stables injectables conditionnées en ampoules stériles, caractérisé en ce qu'on dissout dans du sérum physiologique ou de l'eau pour solutions injectables, une quantité prédéterminée d'alloxane, on ajoute une quantité de 5 à 25 fois plus grande d'un sucre réducteur choisi parmi le groupe constitué par le lévulose, le glucose et le lactose, on filtre sur membrane filtrante utilisée en bactériologie, puis on introduit le soluté obtenu dans une ampoule de 5 ml qu'on scelle au chalumeau et on stérilise par tyndallisation à 60-70 °C.

5. Procédé de préparation de solutions aqueuses d'alloxane stables injectables conditionnées en ampoules stériles, caractérisé en ce qu'on dissout dans du sérum physiologique ou de l'eau pour solutions injectables, une quantité prédéterminée d'alloxane, on ajoute une quantité de 5 à 25 fois plus grande d'un sucre réducteur choisi parmi le groupe constitué par le lévulose, le glucose et le lactose, on filtre sur membrane filtrante utilisée en bactériologie, puis on introduit en atmosphère stérile le soluté obtenu dans une ampoule de 5 ml que l'on scelle au chalumeau.

6. Procédé de préparation de solutions aqueuses d'alloxane stables injectables conditionnées en ampoules stériles, selon la revendication 5, caractérisé en ce qu'on dissout dans de l'eau pour solutions injectables 10 mg d'alloxane pur anhydre et 50 mg de lévulose par ml d'eau.

## Claims

1. Stable aqueous alloxane solutions characterised in that they contain per ml of physiological serum or water for injectable solutions, a predetermined quantity of alloxane and a quantity from 5 to 25 times larger of a reducing sugar selected from the group consisting of laevulose, glucose and lactose.

2. Stable aqueous alloxane solutions according to claim 1, characterised in that they contain per ml of physiological serum or water for injectable solutions, 10 mg of alloxane and 50 mg of laevulose.

3. A process for the preparation of stable aqueous alloxane solutions, characterised in that a predetermined quantity of alloxane is dissolved in the physiological serum or water for injectable solutions and that a reducing sugar is added to this solution in a quantity from 5 to 25 times larger by weight than the said alloxane quantity, the reducing sugar being selected from the group consisting of laevulose, glucose and lactose.

4. A process for the preparation of injectable stable aqueous alloxane solutions packed into sterile ampules, characterised in that a predetermined quantity of alloxane is dissolved in the physiological serum or water for injectable solutions, a quantity from 5 to 25 times larger of a reducing sugar selected from the group consisting of laevulose, glucose and lactose is added, filtering is carried out on filtering membrane used in bacteriology, then the obtained solute is introduced into a 5 ml ampule which is sealed with a tipping torch and is sterilised by tyndallization at from 60 to 70 °C.

5. A process for the preparation of injectable stable aqueous alloxane solutions packed into sterile ampules, characterised in that a predetermined quantity of alloxane is dissolved in the physiological serum or water for injectable solutions, a quantity from 5 to 25 times larger of a reducing sugar selected from the group consisting of laevulose, glucose and lactose is added, filtering is carried out on filtering membrane used in bacteriology, then the obtained solute is introduced in a sterile atmosphere into a 5 ml ampule which is sealed with a tipping torch.

6. A process for the preparation of injectable stable aqueous alloxane solutions packed into sterile ampules, according to claim 5, characterised in that 10 mg of pure anhydrous alloxane and 50 mg of laevulose per ml of water are dissolved in water for injectable solutions.

**Patentansprüche**

1. Wäßrige stabile Alloxanlösungen, dadurch gekennzeichnet, daß sie pro ml physiologisches Serum oder Wasser für Injektionslösungen eine vorbestimmte Menge an Alloxan und eine 5 bis 25 mal so große Menge eines reduzierenden Zuckers, ausgewählt aus der Gruppe bestehend aus Levulose, Glukose und Lactose enthalten.

2. Wäßrige stabile Alloxanlösungen nach Anspruch 1, dadurch gekennzeichnet, daß sie pro ml physiologisches Serum oder Wasser für Injektionslösungen 10 mg Alloxan und 50 mg Levulose enthalten.

3. Verfahren zur Herstellung von wäßrigen stabilen Alloxanlösungen, dadurch gekennzeichnet, daß man in physiologischem Serum oder Wasser für Injektionslösungen eine vorbestimmte Menge Alloxan löst und daß man zu dieser Lösung einen reduzierenden Zucker in einer Gewichtsmenge, die 5 bis 25 mal so groß ist wie die Menge an Alloxan zugibt, wobei der reduzierende Zucker ausgewählt ist aus der Gruppe bestehend aus Levulose, Glukose und Lactose.

4. Verfahren zur Herstellung von stabilen injizierbaren wäßrigen Alloxanlösungen in sterilen Ampullen, dadurch gekennzeichnet, daß man in physiologischem Serum oder Wasser für Injektionslösungen eine vorbestimmte Menge Alloxan löst, eine 5 bis 25 mal so große Menge eines reduzierenden Zuckers, ausgewählt aus der Gruppe bestehend aus Levulose, Glukose und Lactose, zugibt, über ein in der Bakteriologie verwendetes Membranfilter filtriert, anschließend die erhaltene Lösung in eine Ampulle von 5 ml einbringt, die man mit Hilfe eines Brenners zuschweißt und durch Tyndallisieren auf 60 bis 70 °C sterilisiert.

5. Verfahren zur Herstellung von stabilen injizierbaren wäßrigen Alloxanlösungen in sterilen Ampullen, dadurch gekennzeichnet, daß man in physiologischem Serum oder Wasser für Injektionslösungen eine vorbestimmte Menge Alloxan löst, eine 5 bis 25 mal so große Menge eines reduzierenden Zuckers, ausgewählt aus der Gruppe bestehend aus Levulose, Glukose und Lactose zugibt, über ein in der Bakteriologie verwendetes Membranfilter filtriert und anschließend unter steriler Atmosphäre die erhaltene Lösung in eine 5 ml Ampulle einbringt und diese mit Hilfe eines Brenners zuschweißt.

6. Verfahren zur Herstellung von stabilen injizierbaren wäßrigen Alloxanlösungen in sterilen Ampullen nach Anspruch 5, dadurch gekennzeichnet, daß man in Wasser für Injektionslösungen 10 mg reines wasserfreies Alloxan und 50 mg Levulose je ml Wasser löst.